(19) Europäisches Patentamt / European Patent Office / Office européen des brevets

(11) EP 4 373 583 B1

(12) EUROPEAN PATENT SPECIFICATION

(45) Date of publication and mention of the grant of the patent:
**03.09.2025 Bulletin 2025/36**

(21) Application number: **22750840.5**

(22) Date of filing: **18.07.2022**

(51) International Patent Classification (IPC):
**A61Q 11/00** *(2006.01)*    **A61K 8/19** *(2006.01)*
**A61K 8/73** *(2006.01)*

(52) Cooperative Patent Classification (CPC):
**A61Q 11/00; A61K 8/19; A61K 8/73**

(86) International application number:
**PCT/EP2022/070093**

(87) International publication number:
**WO 2023/001776 (26.01.2023 Gazette 2023/04)**

(54) **AN ORAL CARE COMPOSITION**

MUNDPFLEGEZUSAMMENSETZUNG

COMPOSITION DE SOIN BUCCAL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.07.2021 EP 21186820**

(43) Date of publication of application:
**29.05.2024 Bulletin 2024/22**

(73) Proprietors:
• **Unilever IP Holdings B.V.**
**3013 AL Rotterdam (NL)**
Designated Contracting States:
**AL AT BE BG CH CZ DK EE ES FI FR GR HR HU IS LI LT LU LV MC MK NL NO PL PT RO SE SI SK SM**
• **Unilever Global IP Limited**
**Wirral, Merseyside CH62 4ZD (GB)**
Designated Contracting States:
**CY DE GB IE IT MT RS TR**

(72) Inventor: **JOINER, Andrew**
**6708 WH Wageningen (NL)**

(74) Representative: **Tansley, Sally Elizabeth**
**Unilever Patent Group**
**Bronland 14**
**6708 WH Wageningen (NL)**

(56) References cited:
JP-A- 2009 155 217     JP-A- S56 115 711
US-A- 4 353 890        US-A- 5 788 951
US-A1- 2003 082 111

• DATABASE WPI Week 200948, 1 August 2009 Derwent World Patents Index; AN 2009-L47821, XP002805271
• DATABASE WPI Week 198143, 1 October 1981 Derwent World Patents Index; AN 1981-78647D, XP002805272
• TYE ET AL: "Industrial and non-food uses for carrageenan", CARBOHYDRATE POLYMERS, APPLIED SCIENCE PUBLISHERS , LTD BARKING, GB, vol. 10, no. 4, 1 January 1989 (1989-01-01), pages 259 - 280, XP025957111, ISSN: 0144-8617, [retrieved on 19890101], DOI: 10.1016/0144-8617(89)90066-0

## Description

### Field of the Invention

The present invention relates to an oral care composition for minimising staining of teeth.

### Background of the Invention

Long-lasting whitening of teeth is of considerable interest to consumers. Foods and drinks such as tea, coffee and wine may form dental stains by directly depositing chromogens on the tooth surface. Attraction of materials to the tooth surface plays a critical role in the deposition of extrinsic dental stain. The chromogens in these beverages that are responsible for causing dental stain are known as tannins and are composed of polyphenols such as catechins. These materials generate colour due to the presence of conjugated double bonds.

Traditional tooth whitening methods involve either peroxide bleaching from kit formats or abrasive stain removal from toothpaste formats. These particles are essentially insoluble particles that remove extrinsic stain from the surface of the tooth via abrasion when applied with a brush. The present invention relates to a novel oral care composition that delivers effective levels of stain removal.

WO2015/158639 disclose oral care tablets that contain calcium carbonate and kappa carrageenan, The tablets quickly break down and improve the fresh feel of the mouth.

Oral care compositions containing carrageenans are described in US2003/082111 and JP2009 155217 and JP S56 115711

US2003124065A (P&G) discloses an oral care composition and methods for overall cleaning, whitening and preventing, reducing or removing surface deposited stains on natural teeth and dental prosthesis, the compositions comprising in an orally acceptable carrier at least 0.1% by weight of a water-soluble or water-dispersible copolymer prepared by copolymerizing one or a mixture of vinyl pyrrolidone (VP) monomers with one or a mixture of C1-C19 alkyl carboxylic acid (AC) C2-C12 alkenyl ester monomers; preferably, these compositions further comprise one or a mixture of other oral care agents selected from a water soluble alkali metal or ammonium tripolyphosphate in an amount at least about 0.5% by weight of the composition, an abrasive, preferably a precipitated silica abrasive, in an amount at least about 6% by weight of the composition and a bleaching agent in an amount at least about 0.1% by weight of the composition.

It is an object of the present invention to provide a toothpaste that removes the staining of teeth and increases whitening.

### Description of the Invention

A first aspect of the invention relates to a toothpaste composition comprising from 5 wt% to 45 wt% of a calcium carbonate abrasive and carrageenan consisting of at least 60 wt% of the total level of carrageenan of kappa carrageenan, the remainder of the carrageen being iota carrageenan.

A second aspect of the invention relates to the composition described above for use in whitening and/or removing stains from the teeth.

### Detailed Description of the Invention

Except in the examples, or where otherwise explicitly indicated, all numbers in this description indicating amounts of material or conditions of reaction, physical properties of materials and/or use may optionally be understood as modified by the word "about"

All amounts are by weight of the final composition, unless otherwise specified.

It should be noted that in specifying any ranges of values, any particular upper value can be associated with any particular lower value.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis mutandis.*

Compositions according to the invention comprise carrageenan consisting of at least 60 wt% of the total level of carrageenan of kappa carraggenan, the remainder consisting of iota carrageenan preferably the level of kappa carrageenan is greater than 70 wt% of the total level of carrageenan.

The total level of carrageenan is from 0.1 to 5 wt%, preferably 0.3 to 3 wt% of the total composition. Composition of the invention comprise a calcium carbonate abrasive, the calcium carbonate being a particulate material which acts to mechanically remove debris from a tooth surface and debride plaque.

Suitable particulate calcium carbonates may be characterised by the specific shape and size of their constituent primary particles.

By "primary particles" is meant individual particles, defined as the smallest discrete particles that can be seen by electron microscopy analysis (such as, for example, individual crystals).

The primary particles may associate under certain conditions to form larger secondary structures such as aggregates or agglomerates.

For the purposes of the present invention, a suitable source of particulate calcium carbonate includes crystalline calcium carbonates.

The term "crystalline" (in the context of particulate calcium carbonate) generally means a particulate calcium carbonate in which at least 50% by weight, preferably at least 75% by weight, more preferably at least 90% by weight, most preferably more than 95% by weight and ideally more than 99% by weight of the calcium carbonate particles are in the form of crystals.

The term "crystal" means an essentially fully dense solid composed of atoms arranged in an orderly repetitive array bounded by plane surfaces which are the external expression of internal structure. Crystals may be identified and characterised by standard techniques known to those skilled in the art such as X-ray diffraction.

Crystalline calcium carbonates suitable for use in the invention are available naturally (through the extraction and processing of natural ores) or synthetically (through chemical precipitation). There are three main crystalline polymorphs: calcite, aragonite, and less commonly found, vaterite. The vaterite form of calcium carbonate is metastable and irreversibly transforms into calcite and aragonite. There are many different morphologies(crystal habits) for each of these crystalline forms. The calcite crystalline polymorph is the most commonly used crystal form of calcium carbonate. Over 300 crystalline forms of calcite have been reported in the literature.

Particulate calcium carbonates suitable for use in the invention (such as the materials described above) have an average particle size d(50) which can vary over a wide range. Usually the average particle size d(50) is 50 microns or less.

Particle (e.g. crystal) size may be determined by standard techniques known to those skilled in the art such as sedimentation. For the present invention the particle size should be determined using a Malvern mastersizer.

Good cleaning performance has been obtained with crystalline calcium carbonates in which at least 50% by weight, preferably at least 80% by weight of the crystals are rhombohedral calcite crystals with an average particle size d(50) of 30 microns or less, preferably15 microns or less. Most preferable are crystalline calcium carbonates with an average particle size from 0.1 to 30 microns, preferably 0.1 to 15 microns, most preferably 2 to 10 microns (measured using a Malvern mastersizer).

Suitable sources of crystalline calcium carbonate of the size and shape defined above include natural ground calcium carbonate abrasives, generally obtainable from mining and mechanical grinding of sedimentary rocks such as limestone or chalk, or metamorphic rocks such as marble. Natural calcium carbonate is usually of the calcitic polymorph, with a crystal morphology which may typically be characterised as rhombohedral.

Preferred natural ground calcium carbonate abrasives of the type defined above are selected from ground limestone, chalk or marble, optionally refined or partially refined to remove impurities, and having an average particle size ("median ESD" as described above d(50)) of about 30 microns or less, more preferably ranging from about 1 to 15 microns, and most preferably ranging from about 2 to 10 microns. Commercially available examples include those materials sold by the company OMYA™ AG under the names OMYACARB™ 2-AV and OMYACARB™ 5-AV. These are fine ground high purity white marble abrasives having an average particle size ("median ESD" as described above) of about 2 and about 5 microns respectively.

Other types of particulate calcium carbonate may also be suitable, depending on the particular balance of cleaning and abrasion required from a consumer perspective.

Mixtures of any of the above described abrasive cleaning agents may also be used.

The total amount of calcium carbonate in the composition ranges from 5 to 45%, preferably from 5% to 30%, more preferably from 5% to 20% by weight by total weight of the composition.

Suitable foaming agents for use in forming the composition include surfactants. Surfactants help to increase the rate of dissolution of the solid oral care composition when in contact with saliva, and assist in foaming of the composition during usage.

Suitable surfactants include anionic surfactants, such as the sodium, magnesium, ammonium or ethanolamine salts of $C_8$ to $C_{18}$ alkyl sulphates (for example sodium lauryl sulphate), $C_8$ to $C_{18}$ alkyl sulphosuccinates (for example dioctyl sodium sulphosuccinate), $C_8$ to $C_{18}$ alkyl sulphoacetates (such as sodium lauryl sulphoacetate), $C_8$ to $C_{18}$ alkyl sarcosinates (such as sodium lauryl sarcosinate), $C_8$ to $C_{18}$ alkyl phosphates (which can optionally comprise up to 10 ethylene oxide and/or propylene oxide units) and sulphated monoglycerides. Other suitable surfactants include nonionic surfactants, such as optionally polyethoxylated fatty acid sorbitan esters, ethoxylated fatty acids, esters of polyethylene glycol, ethoxylates of fatty acid monoglycerides and diglycerides, and ethylene oxide/propylene oxide block polymers.

Other suitable surfactants include amphoteric surfactants, such as betaines or sulphobetaines.

Preferred surfactants for use in the invention include those anhydrous surfactants selected from sodium lauryl sulfate, sodium lauryl sulfoacetate, cocamidopropyl betaine, sodium alpha olefin sulfonate, dioctyl sodium sulfosuccinate, sodium dodecyl benzene sulfonate and mixtures thereof. Mixtures of any of the above described materials may also be used.

The total amount of surfactant incorporated into the composition of the invention generally ranges from about 0.2 to about 5%, by total weight surfactant based on the total weight of the composition. Compositions of the invention may comprise an additional silica abrasive, but preferably it is not present. Low refractive index silicas, used as abrasives in the present invention are preferably silicas with an apparent refractive index (R.I.) in the range of 1.41 - 1.47, preferably 1.435 - 1.445, preferably having a weight mean particle size of between 5 and 15 mm, a BET (nitrogen) surface area of between 10 and 100 $m^2$/g and an oil absorption of about 70 - 150 $cm^3$/100 g, but abrasive silicas with a lower apparent refractive index may also be used. Typical examples of suitable low refractive index abrasive silicas (e.g. having an R.I. of between 1.435 and 1.445) are Tixosil 63 and 73 ex Rhone Poulenc; Sident 10 ex Degussa; Zeodent 113 ex Zeofinn; Zeodent 124 ex Huber, Sorbosil AC 77 ex PQ Corporation (having an R.I. of approximately 1.440). The amount of these silicas in the composition generally ranges from 5-60% by weight, usually 5-20% by weight.

Compositions according to the invention may comprise a whitening agent. The whitening agent preferably comprises a green and/or a blue pigment. In the context of the present invention a pigment is generally understood to be a shade/material which is insoluble in the relevant medium, at the relevant temperature. This is in contrast to dyes which are soluble. In the context of this invention, the "relevant medium" is human saliva, the liquid medium in which the composition is used, at the temperature of the oral cavity during brushing of the teeth, i.e. up to 37 Degrees C. As a reasonable approximation, the relevant medium may be considered to be water and the relevant temperature to be 25 Degrees C.

Preferably the blue pigment is Pigment Blue 15, more preferably Pigment Blue 15:1, 15:2, 15:3, 15:4, 15:5 or 15:6, most preferably 15:1. A preferred pigment is blue pigment is Phthalocyanine Blue Pigment, CI No. 74160, blue covarine.

The preferred Green pigment is Phthalocyanine Green, preferably Phthalocyanine Green CI-74260.

In one preferred embodiment of the invention the whitening system comprises a combination of green and blue pigment, the weight ratio of green pigment to blue pigment is greater than 1:2, preferably greater than 2:3 most preferably the weight ratio of green pigment to blue pigment is from 2:3 to 3:2.

Preferably the total level of pigment in the composition is from 0.01 wt% to 3 wt, more preferably from 0.02 to 2 wt%.

If the composition is a toothpaste it may be a dual phase paste, if present, the whitening pigments present in one phase.

Compositions according to the invention preferably comprise a polymeric deposition aid. Preferably the composition comprises acid anhydride polymers, particularly preferred are co-polymers of maleic anhydride with methyl vinylether, in which the anhydride moiety may be in a partially or fully hydrolysed or alcoholysed form. Preferred copolymers include Gantrez(R) polymers such as:

> Gantrez S-95: molecular weight 216,000; free acid;
> Gantrez S-96: molecular weight 700,000; free acid;
> Gantrez S-97: molecular weight 1,500,000; free acid; and
> Gantrez MS-955: molecular weight 1,060,000; calcium/sodium salt.
> Particularly preferred co-polymers of maleic acid and methyl vinylether have a molecular weight of 1,000,000 or greater and an especially preferred material is Gantrez S-97.

Compositions of the invention are preferably toothpastes.

The composition, particularly if a toothpaste may comprise an inorganic or a natural or synthetic thickener or gelling agent in proportions of about 0.10 to about 15% by weight depending on the material chosen. These proportions of thickeners in the dentifrice compositions of the present invention form an extrudable, shape-retaining product which can be squeezed from a tube onto a toothbrush and will not fall between the bristles of the brush but rather, will substantially maintain its shape thereon. Suitable thickeners or gelling agents useful in the practice of the present invention include inorganic thickening silicas such as amorphous silicas available from Huber Corporation under the trade designation Zeodent 165, Irish moss, tragacanth, and polyvinylpyrrolidone. In the context of the present invention silica is particularly preferred.

Compositions according to the invention preferably comprise a surfactant, more preferably an anionic surfactant. The level of surfactant is preferably from 0.1 to 5 wt% of the total composition, more preferably from, 0.5wt% to 3 wt%.

The toothpaste composition will comprise further ingredients which are common in the art, such as: antimicrobial agents, e.g. chlorhexidine, sanguinarine extract, metronidazole, quaternary ammonium compounds, such as cetylpyridinium chloride cetylpyridium chloride clay complex; bis-guanides, such as chlorhexidine digluconate, hexetidine, octenidine, alexidine; and halogenated bisphenolic compounds, such as 2,2' methylenebis-(4-chloro-6-bromophenol);

anti-inflammatory agents such as ibuprofen, flurbiprofen, aspirin, indomethacin etc.;
anti-caries agents such as sodium- and stannous fluoride, aminefluorides, sodium monofluorophosphate, sodium trimeta phosphate and casein;
plaque buffers such as urea, calcium lactate, calcium glycerophosphate and strontium polyacrylates;
vitamins such as Vitamins A, C and E;
plant extracts;
desensitising agents, e.g. potassium citrate, potassium chloride, potassium tartrate, potassium bicarbonate, potassium oxalate, potassium nitrate and strontium salts;
anti-calculus agents, e.g. alkali-metal pyrophosphates, hypophosphite-containing polymers, organic phosphonates and phosphocitrates etc.;
biomolecules, e.g. bacteriocins, antibodies, enzymes, etc.;
flavours, e.g. peppermint and spearmint oils;
proteinaceous materials such as collagen;
preservatives;
opacifying agents;
colouring agents;
pH-adjusting agents;
sweetening agents;
pharmaceutically acceptable carriers, e.g. starch, sucrose, water or water/alcohol systems etc.;
Humectants such as glycerol, sorbitol, propyleneglycol, xylitol, lactitol etc.;
binders and thickeners such as sodium carboxymethyl-cellulose, hydroxyethyl cellulose (NatrosoKID), xanthan gum, gum arabic etc. as well as synthetic polymers such as polyacrylates and carboxyvinyl polymers such as Carbopol®;
polymeric compounds which can enhance the delivery of active ingredients such as antimicrobial agents can also be included;
buffers and salts to buffer the pH and ionic strength of the oral care composition; and
other optional ingredients that may be included are e.g. bleaching agents such as peroxy compounds e.g. potassium peroxydiphosphate, effervescing systems such as sodium bicarbonate/citric acid systems, colour change systems, and so on.

Example of the invention will now be illustrated by the following non-limiting examples:

## Examples

### Table 1: Base Paste Formulation

| Ingredient | % w/w |
|---|---|
| Water | To 100 |
| Sorbitol | 30.00 |
| Sodium monofluorophosphate | 1.11 |
| Sodium saccharin | 0.27 |
| Chalk abrasive | 35.0 |
| Silica Thickening | 5.5 |
| Monosodium phosphate | 1.0 |
| Carrageenan | 0.55 |
| Sodium lauryl sulphate | 1.80 |
| Sodium bicarbonate | 5.0 |
| Benzyl alcohol | 0.5 |

(continued)

| Ingredient | % w/w |
|---|---|
| Flavour | 1.0 |
| Carrageenan* Example 1 25 wt% iota, 75wt% kappa<br>Example C 50 wt% iota, 50 wt% kappa<br>Example A 75 wt% iota, 25 wt% kappa<br>Example B 100% iota | |

Hydroxyapatite (HAP) discs were cleaned and polished. The baseline **(initial clean)** colour was measured using a chromameter (Minolta CR200 series). Tannic acid (0.5% w/w) dissolved in Milli Q water and Ammonium iron II sulphate hexahydrate (0.5% w/w) dissolved in Milli Q water were prepared and the solutions were mixed in the ratio of 1:1 by volume to form the FT stain solution and used immediately. The FT stain solution was applied to the HAP discs using a small watercolour paintbrush and left to air dry (approximately 15mins). A further 3 to 4 coats of the stain were applied to each disc and allowed to dry for about 30 minutes. Sufficient stain had been applied once the $L^*$ reading was <56. The discs were then left for ~45 mins before remeasuring the colour **(soiled).** The HAP disks were brushed with the toothpastes for 2 minutes and then for an additional 3 minutes to give a total of 5 minutes of brushing at a speed of 150 cycles per min. The HAP discs were removed from the well, rinsed with water and gently dried using a soft tissue and the colour was remeasured.

The % stain removal was calculated for each HAP disc using the equation below and the mean % stain removal determined.

$$\% \text{ stain removal} = \frac{L^* \text{ (cleaned)} - L^* \text{ (soiled)}}{L^* \text{ (initial clean)} - L^* \text{ (soiled)}} \times 100$$

The results are shown in table 2

**Table 2: Mean % Stain removal after 2 and 5 minutes brushing**

| Example | 1 | C | A | B |
|---|---|---|---|---|
| 2min | 71.9 | 68.6 | 64.5 | 60.0 |
| 5min | 84.9 | 81.7 | 81.0 | 75.0 |

The results show that Example 1 according to the invention remove stains more effectively than the comparative Examples (A, B and C).

## Claims

1. An aqueous toothpaste composition comprising from 5 wt% to 45 wt% of the total composition of a calcium carbonate abrasive and a carrageenan consisting of at least 60 wt% of the total level of carrageenan of kappa carrageenan, the remainder of the carrageen being iota carrageenan.

2. A toothpaste composition according to any preceding claim in which the level of carrageenan is from 0.1 to 5 wt%, preferably 0.3 to 3 wt% of the total composition.

3. A toothpaste composition according to any preceding claim that comprises an aqueous base.

4. A composition according to any preceding claim which further comprises a polyhydric alcohol preferably sorbitol or glycerol.

5. An oral care composition according to any preceding claim that further comprises an anionic surfactant.

6. A composition as described in any preceding claim for use in removing the stains from teeth.

7. A composition as described in any one of claims 1 to 5 for use in whitening the teeth.

**Patentansprüche**

1. Wässrige Zahnpastazusammensetzung, umfassend 5 bis 45 Gew.-% der Gesamtzusammensetzung an einem Calciumcarbonat-Schleifmittel und ein Carrageen, bestehend zu mindestens 60 Gew.-% der Gesamtmenge an Carrageen aus Kappa-Carrageen, wobei der Rest des Carrageens Iota-Carrageen ist.

2. Zahnpastazusammensetzung nach einem vorhergehenden Anspruch, worin die Menge des Carrageens 0,1 bis 5 Gew.-%, vorzugsweise 0,3 bis 3 Gew.-% der Gesamtzusammensetzung beträgt.

3. Zahnpastazusammensetzung nach einem vorhergehenden Anspruch, die eine wässrige Basis umfasst.

4. Zahnpastazusammensetzung nach einem vorhergehenden Anspruch, die außerdem einen mehrwertigen Alkohol umfasst, vorzugsweise Sorbit oder Glycerin.

5. Mundpflegezusammensetzung nach einem vorhergehenden Anspruch, die außerdem ein anionisches Tensid umfasst.

6. Zusammensetzung, wie in einem vorhergehenden Anspruch beschrieben, zur Verwendung bei der Entfernung von Zahnverfärbungen.

7. Zusammensetzung, wie in irgendeinem der Ansprüche 1 bis 5 beschrieben, zur Verwendung beim Aufhellen der Zähne.


**Revendications**

1. Composition de pâte dentifrice aqueuse comprenant de 5 % en poids à 45 % en poids de la composition totale d'un abrasif à base de carbonate de calcium et un carraghénane consistant en au moins 60 % en poids de la teneur totale en carraghénane en carraghénane kappa, le reste du carraghénane étant du carraghénane iota.

2. Composition de pâte dentifrice selon l'une quelconque des revendications précédentes, dans laquelle la teneur en carraghénane est de 0,1 à 5 % en poids, de préférence de 0,3 à 3 % en poids de la composition totale.

3. Composition de pâte dentifrice selon l'une quelconque des revendications précédentes, qui comprend une base aqueuse.

4. Composition selon l'une quelconque des revendications précédentes, qui comprend en outre un alcool polyhydroxylé, de préférence le sorbitol ou le glycérol.

5. Composition de soins bucco-dentaires selon l'une quelconque des revendications précédentes, qui comprend en outre un tensioactif anionique.

6. Composition telle que décrite dans l'une quelconque des revendications précédentes, destinée à être utilisée pour éliminer les taches des dents.

7. Composition telle que décrite dans l'une quelconque des revendications 1 à 5, destinée à être utilisée pour blanchir les dents.

**EP 4 373 583 B1**

## REFERENCES CITED IN THE DESCRIPTION

### Patent documents cited in the description

- WO 2015158639 A
- US 2003082111 A
- JP 2009155217 A
- JP S56115711 A
- US 2003124065 A